# EUROPÄISCHE PATENTSCHRIFT

(11) **EP 1 117 329 B1**
(45) Veröffentlichungstag und Bekanntmachung des Hinweises auf die Patenterteilung: **28.04.2004**
(21) Anmeldenummer: 99944273.4
(22) Anmeldetag: 07.07.1999
(51) Int. Cl.: A61B 5/15

(54) **VORRICHTUNG ZUR PERFORATION VON HAUT**
DEVICE FOR PERFORATING SKIN
DISPOSITIF POUR PERFORER LA PEAU

(30) Priorität: 09.07.1998 DE 19830604
(43) Veröffentlichungstag der Anmeldung: 25.07.2001
(73) Patentinhaber: november Aktiengesellschaft Gesellschaft für Molekulare Medizin, 91056 Erlangen (DE)
(72) Erfinder: BERTLING, Wolf, D-91056 Erlangen (DE)
(74) Vertreter: Gassner, Wolfgang, Dr.
(86) Internationale Anmeldenummer: PCT/DE1999/002092
(87) Internationale Veröffentlichungsnummer: WO 2000/002482

(56) Entgegenhaltungen:
- EP-A- 0 255 338
- EP-A- 0 403 873
- DE-A- 4 320 463
- US-A- 5 147 375
- US-A- 5 350 392

## Beschreibung

Die Erfindung betrifft eine Vorrichtung zur Perforation von Haut.

Eine solche Vorrichtung ist nach dem Stand der Technik unter dem Produktnamen "Lanzer-5" der Firma Becton Dickinson, Oxford OX4 3LY, Großbritannien bzw. der EP 0 061 102 bekannt. Die bekannte Vorrichtung ist aus einer Vielzahl von Einzelteilen aufgebaut. Sie hat ferner den Nachteil, daß in einer zur Wiederverwendung vorgesehenen auf das Gehäuse aufschraubbaren Kappe Blut sich einlagern kann. Solche Bluteinlagerungen sind aus hygienischen Gründen nicht akzeptabel. Ferner ist es denkbar, daß durch an der Außenseite der Kappe anhaftende Verunreinigungen beispielsweise Hautkrankheiten übertragen werden.

Bei aus der EP 0 838 195 A1 und der US 4,653,513 bekannten Vorrichtungen erstreckt sich der Lanzenträger vom Gehäuse in eine daran angebrachte Kappe. Zweck dieser Vorrichtungen ist es, Blut in die Kappe einzusaugen. - Solche Vorrichtungen sind aufwendig, da damit ein Unterdruck zum Einsaugen des Bluts erzeugt werden muß. Die Vorrichtungen sind dementsprechend dicht auszuführen.

Die US 4,375,815 offenbart eine automatisch zurückziehbare Lanzeneinrichtung, die als Wegwerfteil ausgeführt ist. Beim Wegwerfen der Einrichtung werden auch Teile verworfen, die aus hygienischen Gesichtspunkten noch verwendbar wären. Die Vorrichtung ist relativ teuer. Sie verursacht eine unnötige Umweltbelastung.

Die den technologischen Hintergrund der Erfindung betreffenden FR 2 508 305, DE 89 00 203 U1, DE-PS 459 483 und DE-PS 355 891 betreffen Vorrichtungen zur Entnahme von Blutproben, bei denen keine abnehmbare Kappe vorgesehen ist.

Das Dokument EP-A-0 403 873 zeigt eine Vorrichtung mit den im Oberbegriff des Anspruchs 1 definierten Merkmalen.

Aufgabe der Erfindung ist es, die Nachteile nach dem Stand der Technik zu beseitigen. Es soll insbesondere eine Vorrichtung zur Perforation von Haut angegeben werden, die möglichst einfach und kostengünstig herstellbar und hygienisch betreibbar ist.

Diese Aufgabe wird durch die Merkmale des Anspruchs 1 gelöst. Zweckmäßige Ausgestaltungen ergeben sich aus den Merkmalen der Ansprüche 2 bis 18.

Nach Maßgabe der Erfindung ist vorgesehen, daß der Lanzenträger mit einem Kolbenförmigen ersten Abschnitt in der Kappe geführt ist. Dadurch ist es möglich, Kappe und Lanzenträger als Einmalartikel auszubilden. Sämtliche mit dem Patienten in Berührung kommenden Teile werden nach der Perforation der Haut verworfen. Die Vorrichtung ist hygienisch betreibbar.

Vorteilhafterweise ist der Lanzenträger rotationssymmetrisch ausgebildet. Am einen Ende des Lanzenträgers können die Lanze und am anderen Ende ein flanschartiger radial umlaufender Vorsprung vorgesehen sein. Ein solcher Lanzenträger ist einfach herstellbar.

Nach einem Ausgestaltungsmerkmal ist der Lanzenträger in einem korrespondierenden zylindrisch ausgebildeten zweiten Abschnitt in der Kappe geführt. Die Kappe weist zweckmäßigerweise eine erste Öffnung zur Verbindung mit dem Gehäuse und eine zweite Öffnung zum Durchtritt der Lanze auf. Die Kappe und/oder der Lanzenträger können aus spritzgegossenem Kunststoff hergestellt sein. Die zweite Öffnung kann mittels eines, vorzugsweise einstückig angespritzten, Kunststoffilms verschlossen sein. Dadurch ist sichergestellt, daß die Lanze vor der Benutzung nicht verunreinigt wird.

Die Kappe kann mittels eines Bajonettverschlusses, einer Rastverbindung oder eines Gewindes am Gehäuse festlegbar sein. Dadurch wird eine einfache Befestigung realisiert.

Das Element ist vorteilhafterweise so ausgestaltet, daß es bei abgenommener Kappe entgegen der Kraft einer Feder in das Gehäuse bis zu einer vorgespannten ersten Position eindrückund dort selbsttätig verrastbar ist. Durch Betätigen mindestens einer Taste kann die Verrastung gelöst werden, so daß das Element in eine zweite Position stellbar ist. Dadurch wird ein besonders einfacher und kostengünstiger Aufbau der Vorrichtung ermöglicht.

In der zweiten Position verrastet oder verklemmt zweckmäßigerweise ein am Element vorgesehenes Rast- oder Klemmelement mit einem dazu korrespondierenden am anderen Ende des Lanzenträgers vorgesehenen Gegenrast- oder Gegenklemmelement; die Lanze durchgreift vorteilhafterweise den Kunststoffilm und steht über einen Umfangsrand der zweiten Öffnung hervor.

Beim Abnehmen der Kappe mit ausgefahrener Lanze vom Gehäuse kann der Lanzenträger bis zu einem Anschlag mittels des Elements zurückgezogen werden. Nachdem der Lanzenträger am Anschlag anliegt, wird die zwischen Rast- bzw. Klemmelement und Gegenrast- bzw. Gegenklemmelement gebildete Verbindung gelöst. Eine Verletzungsgefahr durch eine über den Öffnungsrand der zweiten Öffnung hervorstehende Lanze wird bei der dann zu verwerfenden Kappe vermieden.

Zur weiteren herstellungstechnischen Vereinfachung der Vorrichtung kann auch das Element aus spritzgegossenem Kunststoff hergestellt sein. Zweckmäßigerweise ist am Element in einstückiger Ausbildung eine Rastfeder angespritzt.

Nach einem weiteren Ausgestaltungsmerkmal kann das Element in einem im Gehäuse aufgenommenen Tragelement verschiebbar geführt sein. In diesem Fall ist die Feder vorteilhafterweise gegen einen Boden des Tragelements abgestützt, wobei ein vom Element sich erstreckender Fortsatz einen im Querschnitt dazu korrespondierend ausgebildeten Durchbruch des Bodens durchgreifen und mittels eines schwalbenschwanzählichen Mittels gegen einen Durchtritt durch den Durchbruch gesichert sein kann. Die vorganannten Merkmale erleichtern die Montage der Vorrichtung.

Das Tragelement erstreckt sich zweckmäßigerweise über den Umfangsrand des Gehäuses, so daß die Kappe darauf aufsteckbar ist.

Ausführungsbeispiele der Erfindung werden anhand der Zeichnung näher erläutert. Hierin zeigen:
- Fig. 1: eine schematische Querschnittsansicht einer ersten Vorrichtung mit abgenommener Kappe,
- Fig. 2: eine schematische Querschnittsansicht der Kappe und eine teilweise Seitenansicht des Gehäuses nach Fig. 1 und
- Fig. 3: eine schematische Querschnittsansicht des Gehäuses einer zweiten Vorrichtung.
- Fig. 4a-f: die Funktion einer dritten Vorrichtung in schematischer Querschnittsansicht.

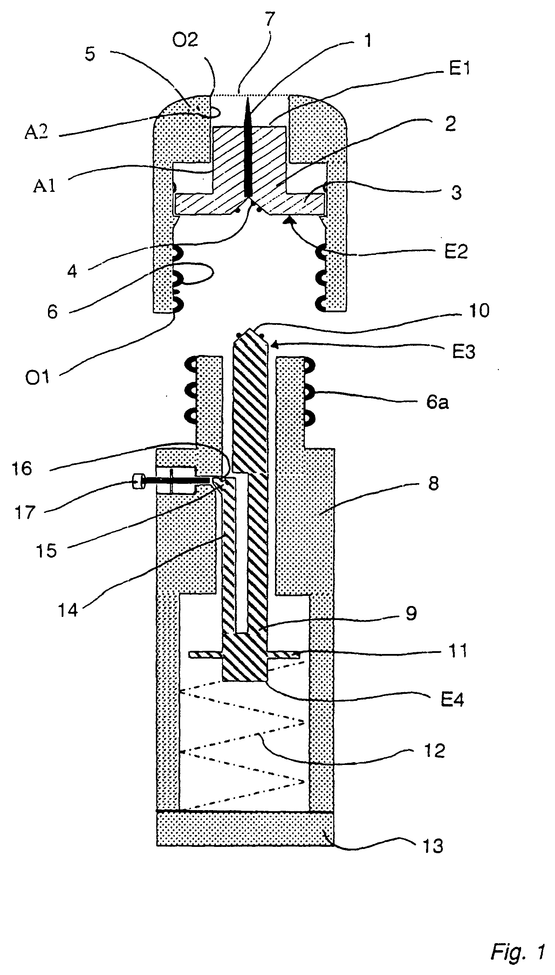

In Fig. 1 ist eine aus Metall hergestellte Lanze 1 am einen Ende E1 eines Lanzenträgers 2 aufgenommen. Der Lanzenträger 2 ist rotationssymmetrisch ausgebildet. Er weist an seinem anderen Ende E2 einen flanschartigen Vorsprung 3 sowie eine V-förmige Ausnehmung 4 auf. Der Lanzenträger 2 ist zweckmäßigerweise aus Kunststoff hergestellt. Er ist mit einem kolbenartigen ersten Abschnitt A1 in einem korrespondierenden zylindrisch ausgebildeten zweiten Abschnitt A2 einer Kappe 5 geführt. In der Nähe einer ersten Öffnung O1 ist an der Innenwand der Kappe 5 ein Innengewinde 6 vorgesehen. Eine zweite Öffnung 02 ist mit einem Kunststoffilm 7 verschlossen. Der Kunststoffilm 7 ist zweckmäßigerweise in einstückiger Ausbildung an die Kappe 5 angespritzt.

In einem Gehäuse 8 ist ein Element 9 verschiebbar aufgenommen. An einem ersten Ende E3 des Elements 9 befindet sich eine zur V-förmigen Ausnehmung 4 korrespondierende Spitze 10.

In der Nähe eines zweiten Endes E4 des Elements 9 ist ein weiterer radial umlaufender Vorsprung 11 vorgesehen. Darauf stützt sich eine Feder 12 ab, die mit ihrem gegenüberliegenden Ende an einem Gehäuseverschluß 13 anliegt. Das Element 9 ist zweckmäßigerweise aus Kunststoff hergestellt. Es weist in einstückiger Ausbildung eine Rastfeder 14 auf. Im hier gezeigten gespannten Zustand greift ein Rastelement 15 in einen am Gehäuse 9 vorgesehenen Durchbruch 16 ein. Das erste Rastelement 15 ist mittels einer Taste 17 niederdrückbar. Ein zum Innengewinde 6 korrespondierendes Außengewinde ist mit 6a bezeichnet.

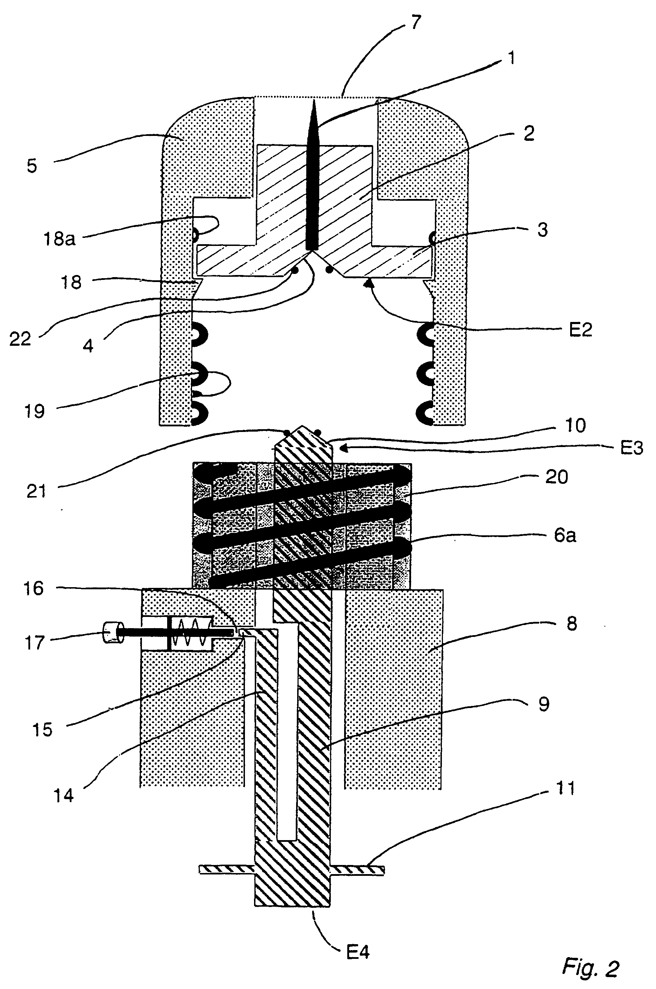

Fig. 2 zeigt die als Einmalartikel vorgesehene Kappe 5 mit darin aufgenommenem Lanzenträger 2. An der Innenwand der Kappe 5 sind mehrere Schnapphaken 18 angespritzt, welche ein Herausfallen des eingesetzten Lanzenträgers 2 verhindern. Erste Noppen 18a halten den Lanzenträger 2 in einer zurückgezogenen Stellung und verhindern ein unbeabsichtigtes Durchstoßen des Kunststoffilms 7. Im Tal eines äußeren Gewindegangs des Innengewindes 6 ist mindestens eine zweite Noppe 19 vorgesehen. - Ein Abschnitt des Gehäuses 8 ist in Seitenansicht gezeigt. Auf dem Kamm eines äußeren Gewindegangs des Außengewindes 6a sind mehrere zur zweiten Noppe 19 korrespondierende Ausnehmungen 20 vorgesehen.

Ein auf der Spitze 10 vorgesehener Wulst 21 ist so ausgebildet, daß er mit in der V-förmigen Ausnehmung angespritzten Fortsätzen 22 verrastbar ist.

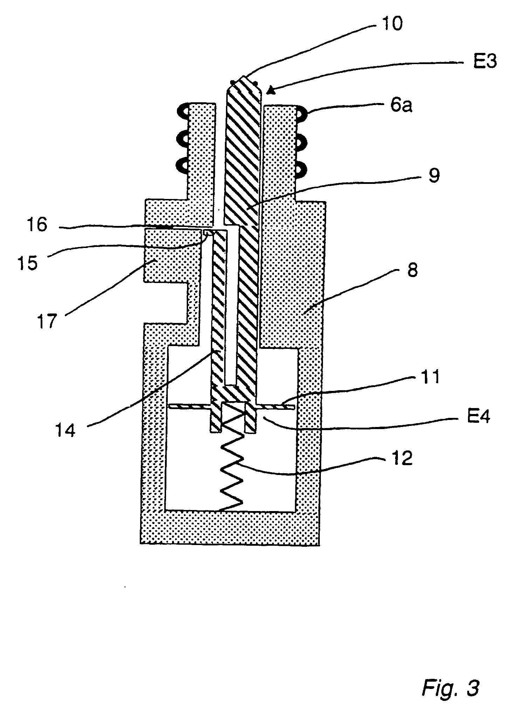

Fig. 3 zeigt das Gehäuse 8 einer zweiten Vorrichtung. Dabei ist der weitere Vorsprung 11 aus flexiblen Zungen gebildet, die zur Montage ein Einschieben des Elements 9 durch die Gehäuseöffnung ermöglichen. Auf das Vorsehen eines Gehäuseverschlusses 13 kann hier verzichtet werden. In weiterer herstellungstechnischer Vereinfachung ist die Taste 17 als einstückig an das Gehäuse 8 angespritztes Federelement ausgebildet.

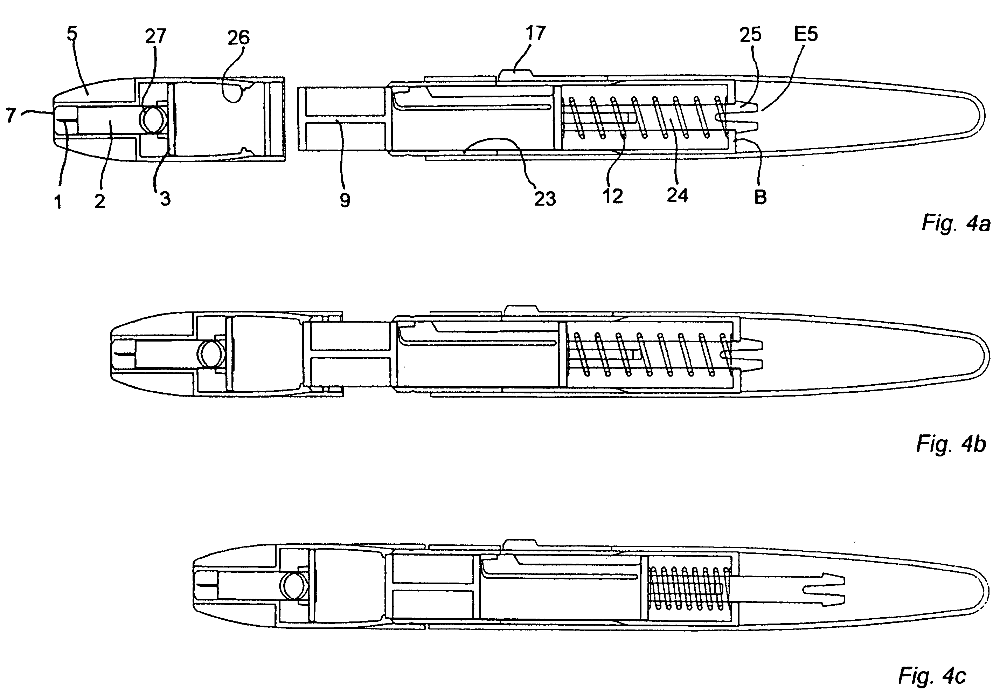

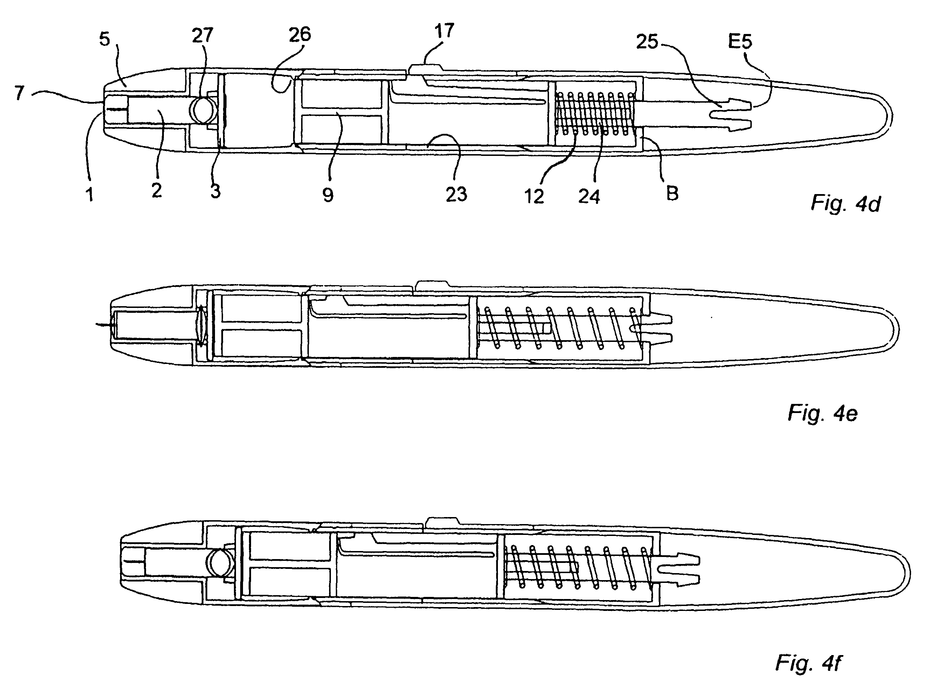

Bei der den Fig. 4a-f gezeigten dritten Vorrichtung ist in das Gehäuse 8 ein Tragelement 23 eingbaut, in dem das Element 9 aufgenommen ist. Vom Element 9 erstreckt sich ein leistenartiger Fortsatz 24. Dessen freies Ende E5 ist als schwalbenschwanzartiges Rastmittel 25 ausgebildet, welches einen im Boden B des Tragelements 23 vorgesehenen weiteren Durchbruch hintergreift. Die Feder 12 ist einerseits gegen den Boden B und andererseits gegen den hier doppelkolbenartig ausgebildeten Lanzenträger 2 abgestützt.

Die Kappe 5 weist mindestens zwei radial nach innen vorspringende weitere Rastfedern 26 auf. Der Lanzernträger 2 ist an der zur Lanze 1 gewandten Seite mit in Form von elastischen Kunststoffringen 27 ausgebildeten Federelementen versehen.

### Die Funktion der Vorrichtungen ist folgende:

Die in den Fig. 1 bis 3 gezeigte Vorrichtung wird durch Eindrücken des Elements 9 entgegen der durch die Feder 12 hervorgerufenen Kraft gespannt. Dabei schnappt das Rastelement 15 in den Durchbruch 16 bzw. hinter eine in der Nähe des Durchbruchs 16 gebildete Kante ein und verhindert so den Wiederaustritt des Elements 9 aus dem Gehäuse 8. Anschließend wird die Kappe 5 auf das Gehäuse 8 aufgeschraubt. Der Austrittsweg der Lanze 1 und damit die Einstichtiefe kann durch die Einschraubtiefe der Kappe 5 am Gehäuse 8 vorgegeben werden. Die vorgegebene Einstichtiefe wird werden durch das Zusammenwirken der zweiten Noppe 19 mit den Ausnehmungen 20 durch ein Einrasten der zweiten Noppe 19 in eine der Ausnehmungen 20 beim Einschrauben der Kappe 8 angezeigt.

Nach dem Einstellen der gewünschten Einstichtiefe wird die Vorrichtung mit der zweiten Öffnung 02 z.B. auf den Finger des Patienten aufgesetzt und die Taste 17 gedrückt. Infolge dessen drückt das Element 9 schlagartig auf den Lanzenträger 2. Die Lanze 1 durchstößt den Kunststoffilm 7 und perforiert die Haut mit der gewünschten Einstichtiefe.

Gleichzeitig verrastet das erste Ende E3 des Elements 9 mit der V-förmigen Ausnehmung 4. Die Verrastung wird dadurch erreicht, daß an der V-förmigen Ausnehmung 4 ein umlaufender Wulst 21 angespritzt ist, der mit Fortsätzen 22 am ersten Ende E3 des Elements 9 korrespondiert. Es ist aber auch denkbar, daß das erste Ende E3 mit einem stumpferen Winkel ausgebildet ist als der Winkel der V-förmigen Ausnehmung 4. In diesem Fall kommt es zu einer klemmenden Verbindung zwischen dem ersten Ende E3 und der V-förmigen Ausnehmung 4.

Nach der Perforation wird die Kappe 5 vom Gehäuse 8 abgeschraubt. Dabei wird der Lanzenträger 2 in seine Ausgangsposition zurückgeschleppt und dort zwischen dem ersten Noppen 18a und den Schnapphaken 18 gehalten. Die Lanze 1 steht dann nicht mehr über die zweite Öffnung 02 hervor. Es kann somit nicht zu unerwünschten Verletzungen kommen. Nach dem Abschrauben der Kappe 5 wird die zwischen dem ersten Ende E3 und der V-förmigem Ausnehmung 4 gebildete Rast- oder Klemmverbindung durch einen leichten Zug gelöst. Die Kappe 5 wird zusammen mit dem Lanzenträger 3 verworfen.

Die Kappe 5 kann auch am Gehäuse 8 verrastet werden. In diesem Fall kann die Einstichtiefe durch die Bereitstellung von Kappen 5 unterschiedlicher Geometrie und/oder Lanzen 1 unterschiedlicher Länge variiert werden.

In den Fig. 4a-f ist die Funktion einer solchen dritten Ausführungsform gezeigt. Beim Aufsetzen der Kappe 5 auf das Gehäuse 8 kommt zunächst das Element 9 mit seinem vorderen Kolben in Anlage mit den weiteren Rastelementen 26. Die Kappe 5 wird dann entgegen der Federkraft der Feder 12 gegen das Gehäuse 8 gedrückt bis das Rastelement 15 in den Durchbruch 16 einrastet. Gleichzeitig werden die weiteren Rastelemente 26 durch den vom Gehäuse 8 überstehenden Teil des Tragelements 23 auseinandergespreizt, so daß das Element 9 gegen den Lanzenträger 2 bewegbar ist. Beim Auslösen des Mechanismus durch Niederdrücken der Taste 17 wird die Lanze 1 schlagartig durch den Kunststoffilm 7 getrieben. Sie wird dann durch die Federwirkung der elastischen Kunststoffringe 27 wieder in die Kappe 5 zurückgezogen, wodurch die Gefahr von Verletzungen an benutzen Kappen gemindert wird.

### Bezugszeichenliste

- 1: Lanze
- 2: Lanzenträger
- 3: Vorsprung
- 4: V-förmige Ausnehmung
- 5: Kappe
- 6: Innengewinde
- 6a: Außengewinde
- 7: Kunststoffilm
- 8: Gehäuse
- 9: Element
- 10: Spitze
- 11: weiterer Vorsprung
- 12: Feder
- 13: Gehäuseverschluß
- 14: Rastfeder
- 15: Rastelement
- 16: Durchbruch
- 17: Taste
- 18: Schnapphaken
- 18a: erste Noppe
- 19: zweite Noppe
- 20: Ausnehmung
- 21: Wulst
- 22: Fortsatz
- 23: Tragelement
- 24: leistenartiger Fortsatz
- 25: Rastmittel
- 26: weiteres Rastelement
- 27: Kunststoffring
- E1: Ende
- E2: anderes Ende
- E3: erstes Ende
- E4: zweites Ende
- E5: freies Ende
- O1: erste Öffnung
- 02: zweite Öffnung
- A1: erster Abschnitt
- A2: zweiter Abschnitt
- B: Boden

## Patentansprüche

1. Vorrichtung zur Perforation von Haut, wobei eine an einem Lanzenträger (2) aufgenommene Lanze (1) mittels eines in einem Gehäuse (8) unter Federvorspannung verrastbaren Elements (9) aus dem Gehäuse (8) ausfahrbar ist, und wobei der Lanzenträger (2) in einer am Gehäuse (8) befestigbaren Kappe (5) verschiebbar aufgenommen ist, **dadurch gekennzeichnet, daß** der Lanzenträger (2) mit einem kolbenförmigen ersten Abschnitt (A1) in der Kappe (5) geführt ist.

2. Vorrichtung nach Anspruch 1, wobei der Lanzenträger (2) rotationssymmetrisch ausgebildet ist.

3. Vorrichtung nach einem der vorhergehenden Ansprüche, wobei am einen Ende (E1) des Lanzenträgers (2) die Lanze (1) und am anderen Ende (E2) ein flanschartiger radial umlaufender Vorsprung (3) vorgesehen sind.

4. Vorrichtung nach einem der vorhergehenden Ansprüche, wobei der Lanzenträger (2) in einem korrespondierenden zylindrisch ausgebildeten zweiten Abschnitt (A2) in der Kappe (5) geführt ist.

5. Vorrichtung nach einem der vorhergehenden Ansprüche, wobei die Kappe (5) eine erste Öffnung (O1) zur Verbindung mit dem Gehäuse (8) und eine zweite Öffnung (02) zum Durchtritt der Lanze (1) aufweist.

6. Vorrichtung nach einem der vorhergehenden Ansprüche, wobei die Kappe (5) und/oder der Lanzenträger (2) aus spritzgegossenem Kunststoff hergestellt ist/sind.

7. Vorrichtung nach einem der vorhergehenden Ansprüche, wobei die zweite Öffnung (02) mittels eines, vorzugsweise einstückig angespritzten, Kunststoffilms (7) verschlossen ist.

8. Vorrichtung nach einem der vorhergehenden Ansprüche, wobei die Kappe (5) mittels eines Bajonettverschlusses, einer Rastverbindung oder eines Gewindes (6, 6a) am Gehäuse (8) festlegbar ist.

9. Vorrichtung nach einem der vorhergehenden Ansprüche, wobei das Element (9) bei abgenommener Kappe (5) entgegen der Kraft einer Feder (12) in das Gehäuse (8) bis zu einer vorgespannten ersten Position eindrückund dort selbststätig verrastbar ist.

10. Vorrichtung nach einem der vorhergehenden Ansprüche, wobei durch Betätigen mindestens einer Taste (17) die Verrastung lösbar ist, so daß das Element (9) in eine zweite Position stellbar ist.

11. Vorrichtung nach einem der vorhergehenden Ansprüche, wobei in der zweiten Position ein am Element vorgesehenes Rast- oder Klemmelement (10, 22) mit einem korrespondierenden am anderen Ende (E2) des Lanzenträgers (2) vorgesehenen Gegenrast- oder Gegenklemmelement (4, 21) verrastet oder verklemmt ist, die Lanze (1) den Kunststoffilm (7) durchgreift und über einen Umfangsrand der zweiten Öffnung (02) hervorsteht.

12. Vorrichtung nach einem der vorhergehenden Ansprüche, wobei beim Abnehmen der Kappe (5) mit ausgefahrener Lanze (1) vom Gehäuse (8) der Lanzenträger (2) bis zu einem Anschlag (18) mittels des Elements (9) zurückziehbar ist.

13. Vorrichtung nach einem der vorhergehenden Ansprüche, wobei das Element (9) aus spritzgegossenem Kunststoff hergestellt ist.

14. Vorrichtung nach einem der vorhergehenden Ansprüche, wobei am Element (9) in einstückiger Ausbildung eine Rastfeder (14, 15) angespritzt ist.

15. Vorrichtung nach einem der vorhergehenden Ansprüche, wobei das Element (9) in einem im Gehäuse (8) aufgenommenen Tragelement (23) verschiebbar geführt ist.

16. Vorrichtung nach Anspruch 15, wobei die Feder (12) gegen einen Boden (B) des Tragelements (23) abgestützt ist.

17. Vorrichtung nach Anspruch 15 oder 16, wobei ein vom Element (9) sich erstreckender Fortsatz (24) einen im Querschnitt dazu korrespondierend ausgebildeten Durchbruch im Boden (B) des Tragelements (23) durchgreift und mittels eines schwalbenschwanzählichen Rastmittels (25) gegen einen Durchtritt durch den Durchbruch gesichert ist.

18. Vorrichtung nach einem der vorhergehenden Ansprüche, wobei das Tragelement (23) sich über den Umfangsrand des Gehäuses (8) erstreckt, so daß die Kappe (5) darauf aufsteckbar ist.

## Claims

1. Device for perforation of skin, wherein a lance (1) held on a lance carrier (2) can be extended from a housing (8) via an element (9) which can be snapped in a housing (8) under spring pre-tension, and wherein the lance carrier (2) is located slideably in a cap (5) secured on the housing (8), **characterised in that** the lance carrier (2) is guided with a piston-shaped first section (A1) in the cap (5).

2. Device as defined in claim 1, wherein the lance carrier (2) is formed rotation-symmetrically.

3. Device as defined in one of the preceding claims, wherein the lance (1) is provided on one end (E1) of the lance carrier (2) and a flange-type, radial circumferential projection (3) is provided on the other end (E2).

4. Device as defined in one of the preceding claims, wherein the lance carrier (2) is guided in a corresponding, cylindrically formed second section (A2) in the cap (5).

5. Device as defined in one of the preceding claims, wherein the cap (5) has a first opening (O1) for connection with the housing (8) and a second opening (02) for penetration of the lance (1).

6. Device as defined in one of the preceding claims, wherein the cap (5) and/or the lance carrier (2) is/are made of injected-moulded plastic.

7. Device as defined in one of the preceding claims, wherein the second opening (02) is locked via a preferably one-piece, injection-moulded plastic film (7).

8. Device as defined in one of the preceding claims, wherein the cap (5) can be fixed via a bayonet catch, a snap-in connection or a thread (6, 6a) on the housing (8).

9. Device as defined in one of the preceding claims, wherein, when the cap (5) is removed, the element (9) can be pressed against the force of a spring (12) into the housing (8) up to a pre-tensioned first position and automatically snapped in there.

10. Device as defined in one of the preceding claims, wherein snap-in position can be released by pressing at least one key (17) so that the element (9) can be set in a second position.

11. Device as defined in one of the preceding claims, wherein, in the second position, a snap or clamping element (10, 22) provided on the element with a corresponding counter snap or counter clamping element (4, 21) provided on the other end (E2) of the lance carrier (2) is snapped in or clamped, the lance (1) passes through the plastic film (7) and protrudes over a circumferential edge of the second opening (O2).

12. Device as defined in one of the preceding claims, wherein, when the cap (5) is being removed with lance (1) extended from the housing (8), the lance carrier (2) can be pulled back up to a stop (18) via the element (9).

13. Device as defined in one of the preceding claims, wherein the element (9) is made of injection-molded plastic.

14. Device as defined in one of the preceding claims, wherein a snap-in spring (14, 15) is injection-moulded on the element (9) in a one-piece form.

15. Device as defined in one of the preceding claims, wherein the element (9) is positioned in a carrying element (23) located in the housing (8) which carrying element can be moved.

16. Device as defined in claim 15, wherein the spring (12) is supported against a floor (B) of the carrying element (23).

17. Device as defined in claim 15 or 16, wherein a prolongation (24) extending from the element (9) passes through a breakthrough in the floor (B) of the carrying element (23) which breakthrough is correspondingly thereto formed in the cross section, and is secured via a dovetail-like snap element (25) against penetration through the breakthrough.

18. Device as defined in one of the preceding claims, wherein the carrying element (23) extends over the circumferential edge of the housing (8) so that the cap (5) can be placed thereon.

## Revendications

1. Dispositif destiné à la perforation de la peau, une lancette (1) disposée dans un porte-lancette (2) pouvant être sortie du boîtier (8) à l'aide d'un élément (9) enclenchable sous tension de ressort dans un boîtier (8) et le porte-lancette (2) pouvant coulisser dans un capuchon (5) fixé au boîtier (8), **caractérisé par le fait que** le porte-lancette (2), avec une première partie en forme de piston (A1), est amené dans le capuchon (5).

2. Dispositif selon la revendication 1, le porte-lancette (2) étant tel qu'il soit à symétrie de révolution.

3. Dispositif selon l'une des revendications précédentes, la lancette (1) étant prévue à une extrémité (E1) du porte-lancette (2) et une saillie (3), en forme de bride, circulaire par rapport à un axe radial, étant prévue à l'autre extrémité (E2).

4. Dispositif selon l'une des revendications précédentes, le porte-lancette (2), avec une deuxième partie (A2) correspondante en forme de cylindre, étant amené dans le capuchon (5).

5. Dispositif selon l'une des revendications précédentes, le capuchon (5) présentant une première ouverture (O1) destinée à la liaison avec le boîtier (8) et une deuxième ouverture (02) pour la sortie de la lancette (1).

6. Dispositif selon l'une des revendications précédentes, le capuchon (5) et/ou le porte-lancette (2) étant en plastique moulé par injection.

7. Dispositif selon l'une des revendications précédentes, la deuxième ouverture (02) étant obturée à l'aide d'un film plastique (7), moulé d'une pièce de préférence.

8. Dispositif selon l'une des revendications précédentes, le capuchon (5) pouvant être fixé au boîtier (8) à l'aide d'un joint à baïonnette, d'un élément encliquetable ou d'un filetage (6, 6a).

9. Dispositif selon l'une des revendications précédentes, l'élément (9), capuchon (5) retiré, pouvant être enfoncé et enclenché automatiquement dans le boîtier (8) jusqu'à une première position précontrainte, sens opposé à la tension d'un ressort (12).

10. Dispositif selon l'une des revendications précédentes, l'enclenchement pouvant être déclenché en appuyant au moins sur une touche (17) de façon à ce que l'élément (9) soit réglable dans une deuxième position.

11. Dispositif selon l'une des revendications précédentes, un élément d'enclenchement ou de serrage (10, 22), dans la deuxième position, étant enclenché ou serré sur l'élément avec un élément correspondant de contre-enclenchement ou de contre-serrage (4, 21) prévu sur l'autre extrémité (E2) du porte-lancette (2). La lancette (1) pénétrant dans le film plastique (7) et dépassant du bord circonférentiel de la deuxième ouverture (02).

12. Dispositif selon l'une des revendications précédentes, le porte-lancette (2) pouvant être retiré jusqu'à une butée (18) à l'aide de l'élément (9) en retirant le capuchon (5) du boîtier (8), lancette (1) sortie.

13. Dispositif selon l'une des revendications précédentes, l'élément (9) étant en matière plastique moulée par injection.

14. Dispositif selon l'une des revendications précédentes, un ressort à cran d'arrêt (14, 15) étant moulé sur l'élément (9) en une pièce.

15. Dispositif selon l'une des revendications précédentes, l'élément (9) étant coulissant dans un élément porteur (23) logé dans le boîtier (8).

16. Dispositif selon la revendication 15, le ressort (12) étant appuyé contre un fond (B) de l'élément porteur (23).

17. Dispositif selon la revendication 15 ou 16, un prolongement (24) de l'élément (9) pénétrant transversalement dans le passage correspondant formé par le fond (B) de l'élément porteur (23) et étant sécurisé par un élément d'arrêt (25) en forme de queue d'aronde contre une sortie par le passage.

18. Dispositif selon l'une des revendications précédentes, l'élément porteur (23) s'étendant au-delà du bord circonférentiel du boîtier (8) de façon à pouvoir y placer le capuchon (5).
